# EUROPEAN PATENT APPLICATION

(11) **EP 3 549 459 A1**
(43) Date of publication of application: **09.10.2019**
(21) Application number: 17876471.8
(22) Date of filing: 08.05.2017
(51) Int. Cl.: A24F 47/00

(54) **CARTRIDGE AND ATOMIZER THEREOF, AND ELECTRONIC CIGARETTE**

(30) Priority: 02.12.2016 CN 201611101786
(71) Applicant: Changzhou Jwei Intelligent Technology Co., Ltd., Changzhou, Jiangsu 213125 (CN); Joyetech Europe Holding GmbH, 6303 Zug (CH)
(72) Inventor: QIU, Weihua, Jiangsu 213125 (CN)
(74) Representative: Zaboliene, Reda
(86) International application number: PCT/CN2017/083506
(87) International publication number: WO 2018/098995

(57) **Abstract**

Disclosed are a cartridge (10) and an electronic cigarette (100) having the cartridge (10). The cartridge (10) comprises a cartridge carrier (11) having a liquid storage cavity (114), a liquid inlet member (13) accommodated inside the cartridge carrier (11), an adjustment member (14), a cartridge base (15) arranged at one end of the cartridge carrier (11), and an elastic member (16) accommodated in the cartridge base (15). The liquid inlet member (13) is provided with a second liquid inlet hole (1312) in communication with the liquid storage cavity (114). The adjustment member (14) is provided with a communication portion (142) and a sealing portion (143). The adjustment member (14) is slidably arranged between the liquid inlet member (13) and the cartridge base (15). One end of the elastic member (16) elastically abuts against the adjustment member (14), and the other end of the elastic member (16) elastically abuts against the cartridge base (15) so as to enable the sealing portion (143) to close the second liquid inlet hole (1312). The sliding of the adjustment member (14) enables the adjustment member (14) to move away from the liquid inlet member (13), so that the communication portion (142) can be in communication with the second liquid inlet hole (1312). The cartridge (10) controls open and closed states of the second liquid inlet hole (1312) by means of sliding and changing the position of the adjustment member (14) and therefore has a good child protection function, and has a simple structure and is convenient to operate.

## Description

### TECHNICAL FIELD

The present application relates to the technical field of smoking simulation, and more particularly, to a cartridge, an atomizer, and an electronic cigarette.

### BACKGROUND

Current electronic cigarettes generally have a split type structure, the liquid storage assembly and the atomizing assembly are connected to each other by a pluggable structure or a threaded structure. When the atomizing assembly is detached from the liquid storage assembly, the inlet or outlet hole of the liquid storage assembly for the tobacco liquid is always in an open state, thus the tobacco liquid stored in the liquid storage assembly is easy to leak out. When this kind of electronic cigarette is accessed by children, the liquid storage assembly and the atomizing assembly may be detached from each other by merely a simple operation such as plugging or rotation, thus children may contact or ingest the tobacco liquid by accident, it has a potential safety hazard for children's health.

### SUMMARY

Accordingly, it is necessary to provide a cartridge having a children protection function, and an atomizer and an electronic cigarette having the cartridge.

The technical solution adopted by the present application to solve the problem is as follows.

A cartridge includes a cartridge carrier having a liquid storage chamber, a liquid inlet member and an adjusting member received in the cartridge carrier, a cartridge base disposed at one end of the cartridge carrier, and an elastic member received in the cartridge base. The liquid inlet member is provided with a second liquid inlet hole in communication with the liquid storage chamber. The adjusting member is provided with a communication portion and a sealing portion. The adjusting member is slidably disposed between the liquid inlet member and the cartridge base. One end of the elastic member elastically resists the adjusting member, the other end of the elastic member elastically resists the cartridge base to cause the sealing portion to seal the second liquid inlet hole. When the adjusting member is brought to slide in a direction away from the liquid inlet member, the communication portion is in communication with the second liquid inlet hole.

Further, an outer wall of the adjusting member is provided with at least one guiding protrusion along an axial direction of the adjusting member, and an inner wall of the cartridge base is provided, corresponding to the guiding protrusion, with at least one guiding groove along an axial direction of the cartridge base. Or optionally, an outer wall of the adjusting member is provided with at least one guiding groove along an axial direction of the adjusting member, and an inner wall of the cartridge base is provided, corresponding to the guiding groove, with at least one guiding protrusion protruding inwardly along an axial direction of the cartridge base. The guiding protrusion is capable of sliding along the guiding groove.

Further, the inner wall of the cartridge base is provided with an abutting portion at one end away from the cartridge carrier, and the abutting portion protrudes radially inwardly along a circumferential direction of the cartridge base. One end of the elastic member elastically resists the abutting portion, the other end of the elastic member elastically resists the adjusting member at one end away from the liquid inlet member.

Further, the cartridge carrier includes a liquid storage tube and an exhaust tube extending through the liquid storage tube along an axial direction of the liquid storage tube. The liquid storage chamber is formed by a space between an inner wall of the liquid storage tube and an outer wall of the exhaust tube. An exhaust passage is formed by an interior space of the exhaust tube.

Further, the cartridge further includes a pressing plate received in the cartridge carrier. The pressing plate is fixedly mounted on one end of the liquid inlet member away from the adjusting member. The pressing plate is provided with a first liquid inlet hole corresponding to the second liquid inlet hole. The first liquid inlet hole is in communication with the liquid storage chamber.

An atomizer includes the aforementioned cartridge. The atomizer further includes an atomizing assembly detachably connected to the cartridge. The atomizing assembly includes an atomizing end cover connected to the adjusting member by threads. When the cartridge or the atomizing assembly is rotated, the adjusting member is driven to move towards or away from the liquid inlet member.

Further, an inner wall of the adjusting member is provided with an internal thread. An outer wall of the atomizing end cover is provided with an external thread matching with the internal thread. The external thread of the atomizing end cover extends through the cartridge base and engages with the internal thread.

Further, an atomizing chamber is formed by an interior space of the atomizing end cover. The atomizing end cover is provided with a third liquid inlet hole in communication with the atomizing chamber.

Further, the pressing plate is provided with a first mounting hole, the liquid inlet member is provided with a second mounting hole, the adjusting member is provided with a third mounting hole, and the atomizing end cover is provided with a fourth mounting hole. The exhaust tube extends sequentially through the first mounting hole, the second mounting hole, the third mounting hole and the fourth mounting hole, to enable the atomizing chamber to be in communication with the exhaust passage.

An electronic cigarette includes the aforementioned atomizer, wherein the electronic cigarette further includes a battery assembly connected to the atomizer.

The advantages of the present application are as follows.

Only when the cartridge or the electronic cigarette provided by the present application is in use, the tobacco liquid flows out the cartridge to be atomized. When the cartridge and the atomizing assembly are disassembled and separated, the sealing portion of the adjusting member seals the second liquid inlet hole under the action of the elastic member, and the tobacco liquid is thus sealed. Even if the children contact and open the electronic cigarette, it will not cause harm to the children. Thus, it has a good protection function for children with a simple structure and is convenient to operate.

### BRIEF DESCRIPTION OF THE DRAWINGS

The present application will now be further described with reference to the accompanying drawings and embodiments.
FIG. 1 is a schematic view of an electronic cigarette of the present application;
FIG. 2 is a schematic view of a cartridge of the electronic cigarette of FIG. 1;
FIG. 3 is a schematic view of an atomizing assembly and a battery assembly of the electronic cigarette of FIG. 1;
FIG. 4 is an exploded view of the electronic cigarette of FIG. 1;
FIG. 5 is a schematic view of a cartridge carrier of the electronic cigarette of FIG. 4;
FIG. 6 is a schematic view of a liquid inlet member of the electronic cigarette of FIG. 4;
FIG. 7 is an enlarged view of an adjusting member of the electronic cigarette of FIG. 4;
FIG. 8 is a schematic view of an atomizer base of the electronic cigarette of FIG. 4;
FIG. 9 is an enlarged view of an atomizing end cover of the electronic cigarette of FIG. 4;
FIG. 10 is a cross-sectional view of the electronic cigarette of FIG. 1 (showing a closed state of the second liquid inlet hole);
FIG. 11 is another cross-sectional view of the electronic cigarette (showing an open state of the second liquid inlet hole);
FIG. 12 is another cross-sectional view of the electronic cigarette (rotated 90 degrees relative to FIG. 11).

The part names and their reference signs in the figures are:

| | | |
|---|---|---|
| electronic cigarette 100 | cartridge 10 | atomizing assembly 20 |
| cartridge carrier 11 | pressing plate 12 | liquid inlet member 13 |
| adjusting member 14 | cartridge base 15 | elastic member 16 |
| housing 21 | battery 22 | atomizing base 23 |
| heating device 24 | atomizing end cover 25 | pressure sensor 26 |
| liquid storage tube 112 | exhaust tube 113 | liquid storage chamber 114 |
| exhaust passage 115 | first mounting hole 121 | first liquid inlet hole 122 |
| positioning hole 123 | main body 131 | connecting body 132 |
| third mounting hole 141 | communication portion 142 | sealing portion 143 |
| guiding protrusion 144 | internal thread 145 | guiding groove 151 |
| abutting portion 152 | first restricting protrusion 153 | air intake hole 211 |
| supporting base 231 | electrode fixing bracket 232 | liquid absorbing member 241 |
| heating member 242 | second restricting protrusion 251 | fourth mounting hole 252 |
| third liquid inlet hole 253 | external thread 254 | atomizing chamber 255 |
| resisting portion 1121 | second mounting hole 1311 | second liquid inlet hole 1312 |
| positioning protrusion 1313 | first restricting groove 1314 | air intake groove 2311 |
| through hole 2312 | elongated groove 2313 | second restricting groove 2314 |

### DETAILED DESCRIPTION OF EMBODIMENTS

The present application is specifically illustrated with reference to accompanying drawings. The accompanying drawings are schematic views which simplified shows fundamental structures of an exemplary embodiment of the present application. Thus, merely the constructions related to the present application are shown.

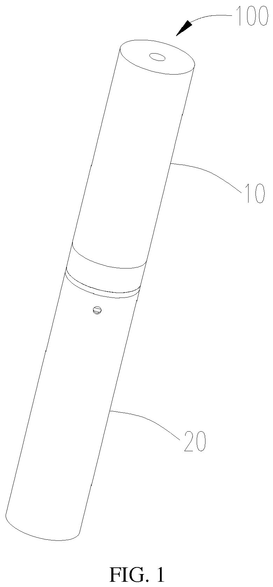

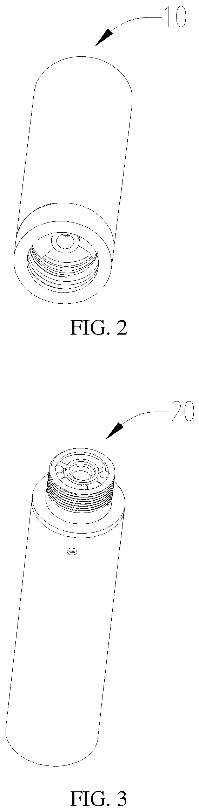

Referring to FIG. 1 to FIG. 3, the present application provides an electronic cigarette 100, the electronic cigarette 100 includes an atomizer (not labeled) and a battery assembly (not labeled). The atomizer includes a cartridge 10 configured for storing tobacco liquid and an atomizing assembly 20 configured for atomizing the tobacco liquid to generate aerosol. The cartridge 10 is detachably connected to the atomizing assembly 20. The battery assembly is detachably connected to the atomizing assembly 20 for driving the atomizing assembly 20 to atomize the tobacco liquid.

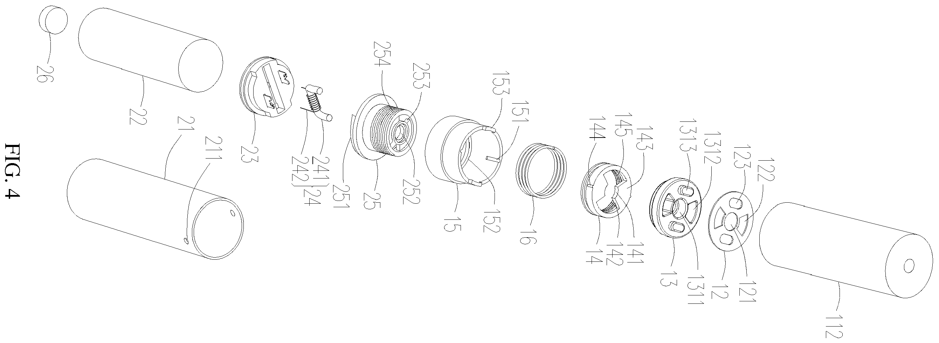

Referring also to FIG. 4, the cartridge 10 includes a cartridge carrier 11, a pressing plate 12, a liquid inlet member 13, an adjusting member 14, an elastic member 16, and a cartridge base 15. The pressing plate 12, the liquid inlet member 13, the adjusting member 14 and the elastic member 16 are received in the cartridge carrier 11. The cartridge base 15 is detachably assembled to one end of the cartridge carrier 11 adjacent to the atomizing assembly 20. The adjusting member 14 is slidably arranged between the liquid inlet member 13 and the cartridge base 15. The elastic member 16 is elastically resisted between the adjusting member 14 and the cartridge base 15.

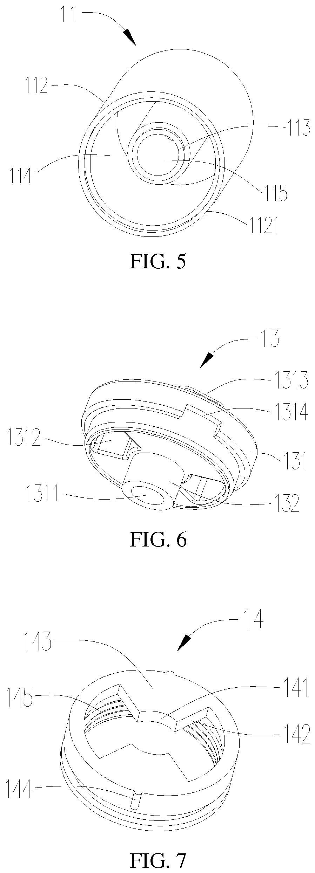

Referring to FIG. 5, the cartridge carrier 11 substantially has a tubular shape. The cartridge carrier 11 includes a liquid storage tube 112 having an opening at an end, and an exhaust tube 113 received in and extending through the liquid storage tube 112. The exhaust tube 113 is positioned at a center of the liquid storage tube 112. A liquid storage chamber 114 for storing tobacco liquid is formed between an inner wall of the liquid storage tube 112 and an outer wall of the exhaust tube 113. An interior space of the exhaust tube 113 forms an exhaust passage 115 for user to inhale. The user can inhale through a top of the exhaust tube 113.

Further, the liquid storage tube 112 forms a resisting portion 1121 on the inner wall of the liquid storage tube 112 adjacent to the opening, wherein the resisting portion 1121 has an annular shape and extends along a circumferential direction of the liquid storage tube 112.

In the embodiment, the liquid container 11 can serve as a mouthpiece directly. The user can inhale by keeping one end of the liquid container 11 in his/her mouth. It can be understood that, in an alternative embodiment, a communication member can be connected to the liquid container 11 for communicating with the exhaust tube 113, and the user can inhale by keeping the communication member in his/her mouth.

The pressing plate 12 substantially has a disc-shaped plate structure. A first mounting hole 121 matching with the exhaust tube 113 is provided at a center of the pressing plate 12. The pressing plate 12 is further provided with a first liquid inlet hole 122 and a positioning hole 123 around the first mounting hole 121. The pressing plate 12 is sleeved on the exhaust tube 113 through the first mounting hole 121. The outer edge of the pressing plate 12 resists on the resisting portion 1121 of the liquid storage tube 112 and is connected to the inner wall of the liquid storage tube 112 tightly.

In the embodiment, the first liquid inlet hole 122 has two and the two first liquid inlet holes 122 are symmetrically arranged about the first mounting hole 121. The positioning hole 123 has two and the two positioning holes 123 are symmetrically arranged about the first mounting hole 121. The first liquid inlet holes 122 and the positioning holes 123 are alternately distributed along a circle. It can be understood that, in alternative embodiments, the first liquid inlet hole 122 and the positioning hole 123 can each have one or more than two.

In the embodiment, the pressing plate 12 is a plastic circular plate. It can be understood that, in alternative embodiments, the pressing plate 12 can also be made of other hard materials such as stainless steel sheet or ceramic sheet.

Referring to FIG. 6, the liquid inlet member 13 substantially has a cylindrical shape. The liquid inlet member 13 includes a main body 131 and a connecting body 132 protruded from a center of the main body 131. The connecting body 132 is positioned on one end of the main body 131 away from the pressing plate 12. The main body 131 is provided with a second mounting hole 1311 at the center, the second mounting hole 1311 extends through the connecting body 132 and matches with the exhaust tube 113. The main body 131 is provided, around the second mounting hole 1311, with a second liquid inlet hole 1312 corresponding to the first liquid inlet hole 122 and a positioning protrusion 1313 corresponding to the positioning hole 123. The main body 131 is further provided with a first restricting groove 1314 at a circumferential surface of the main body 131. The liquid inlet member 13 is sleeved on one end of the exhaust tube 113 through the second mounting hole 1311. When the positioning protrusion 1313 engages in the positioning hole 123, the second liquid inlet hole 1312 is in communication with the first liquid inlet hole 122 of the pressing plate 12. The liquid inlet member 13 is tightly abuts against the inner wall of the liquid storage tube 112. The second mounting hole 1311 is hermetically connected to the outer wall of the exhaust tube 113.

In the embodiment, the liquid inlet member 13 is made of rubber. It can be understood that, the liquid inlet member 13 can also be made of silicone, for providing a sealing function.

Due to a softness of the material of the liquid inlet member 13, the pressing plate 12 closely contacts the liquid inlet member 13 to ensure the liquid inlet member 13 uneasy to be deformed, thereby avoiding a liquid leakage.

In the embodiment, the restricting between the liquid inlet member 13 and the pressing plate 12 is realized by an engagement between the positioning protrusion 1313 and the positioning hole 123. It can be understood that, the pressing plate 12 can be fixed to the liquid inlet member 13 via adhering.

Referring to FIG. 7, the adjusting member 14 substantially has a tubular shape. The adjusting member 14 is positioned on one end of the liquid inlet member 13 adjacent to the atomizing assembly 20. The adjusting member 14 is provided with a third mounting hole 141 corresponding to the connecting body 132 at a center of the adjusting member 14. The adjusting member 14 is provided with a communication portion 142 and a sealing portion 143 around the third mounting hole 141. The adjusting member 14 is sleeved on the connecting body 132 of the liquid inlet member 13 through the third mounting hole 141. When the adjusting member 14 closely contacts one end of the liquid inlet member 13, the sealing portion 143 can seal the second liquid inlet hole 1312 of the liquid inlet member 13.

Further, the adjusting member 14 is provided with two strip-like guiding protrusions 144 which are disposed oppositely to each other. The two guiding protrusions 144 protrude outwardly from an outer wall of the adjusting member 14 along an axial direction of the adjusting member 14. The adjusting member 14 is provided with an internal thread 145 on an inner wall of the adjusting member 14. It can be understood that, the number of the guiding protrusions 144 can be one or more than two, the guiding protrusion 144 can have other shapes such as dot-like bump.

The cartridge base 15 is substantially a hollow cylindrical structure having openings at two ends. The cartridge base 15 is provided, corresponding to the two guiding protrusions 144, with two guiding grooves 151 which are disposed oppositely to each other. The two guiding grooves 151 are defined in the inner wall of the cartridge base 15 along an axial direction of the cartridge base 15. The inner wall of the cartridge base 15 is provided with an abutting portion 152 at one end adjacent to the atomizing assembly 20. The abutting portion 152 protrudes radially inwardly along a circumferential direction of the cartridge base 15. The cartridge base 15 is provided with a first restricting protrusion 153 matching with the first restricting groove 1314. The first restricting protrusion 153 protrudes from one end of the cartridge base 15 away from the atomizing assembly 20 and along an axial direction of the cartridge base 15.

The cartridge base 15 is fixedly assembled to the opening end of the liquid storage tube 112. The guiding protrusion 144 of the adjusting member 14 can slide in the guiding groove 151 of the cartridge base 15. A length of the guiding groove 151 along an axial direction of the cartridge base 15 limits a sliding distance of the adjusting member 14.

It can be understood that, the guiding groove 151 can also be defined in the outer wall of the adjusting member 14 along an axial direction of the adjusting member 14. At this case, the guiding protrusion 144 protrudes outwardly from the inner wall of the cartridge base 15 and extends along an axial direction of the cartridge base 15 corresponding to the guiding groove 151. Similarly, the adjusting member 14 can slide along the guiding groove 151.

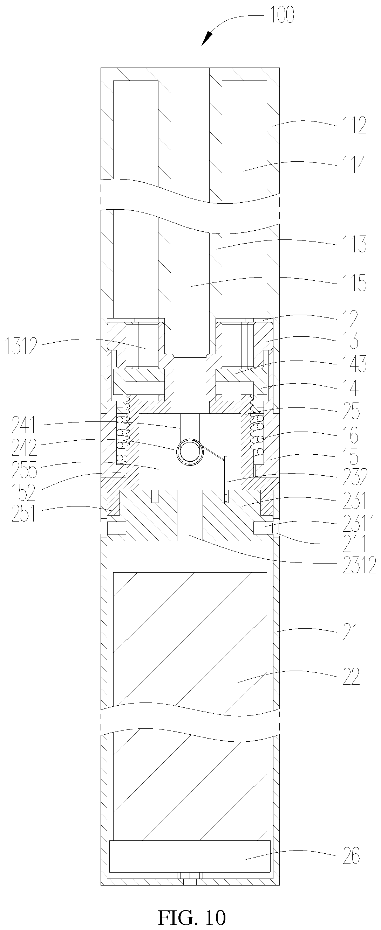

Referring to FIG. 10, one end of the elastic member 16 elastically resists the abutting portion 152, the other end of the elastic member 16 resists the adjusting member 14 at one end adjacent to the atomizing assembly 20. At an initial state, the elastic member 16 tightly presses the adjusting member 14 against the liquid inlet member 13, and the sealing portion 143 of the adjusting member 14 seals the second liquid inlet hole 1312 of the liquid inlet member 13.

In the embodiment, the elastic member 16 is a spring. It can be understood that, the form of the elastic member 16 is not limited. The elastic member 16 may be a member having rigidity and elasticity such as thin steel sheet, which falls into the protection scope of the present application.

Referring to FIG. 4, the atomizing assembly 20 includes a housing 21, an atomizing base 23 received in the housing 21, a heating device 24 disposed on the atomizing base 23, and an atomizing end cover 25 detachably disposed on one end of the housing 21.

The battery assembly includes a battery 22 received in the housing 21 and a controlling device (not shown) for controlling the battery 22 to supply power to the heating device 24.

The housing 21 substantially has a hollow tubular structure with an opening at one end. The tubular wall of the housing 21 is provided with two air intake holes 211 at one end of the housing 21 adjacent to the opening end. The two air intake holes 211 are disposed oppositely and are in communication with an interior of the housing 21. It can be understood that, the number of the air intake hole 211 can also be one or more than two.

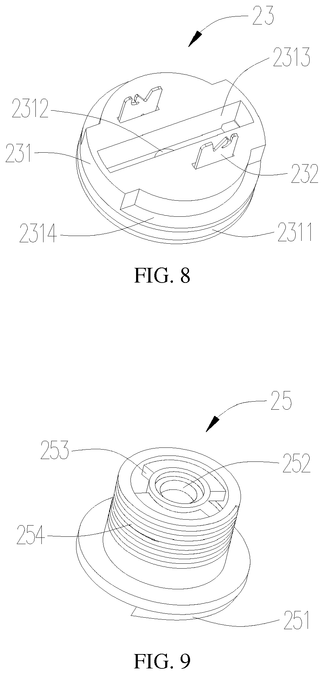

Referring to FIG. 8, the atomizing base 23 has a disc-shaped structure. The atomizing base 23 includes a supporting base 231 closely received in the housing 21 and a pair of electrode fixing brackets 232 disposed on the supporting base 231.

The supporting base 231 is provided with an air intake groove 2311 in the circumferential surface of the supporting base 231. The air intake groove 2311 is in communication with the air intake hole 211. The supporting base 231 is provided with a through hole 2312 at a lower end of the supporting base 231. The supporting base 231 is provided with an elongated groove 2313 at an upper end of the supporting base 231. The elongated groove 2313 is in communication with the air intake groove 2311 and the through hole 2312. The supporting base 231 is further provided with a second restricting groove 2314 at an outer edge of the upper end of the supporting base 231.

The electrode fixing bracket 232 extends through the supporting base 231. The pair of electrode fixing brackets 232 are respectively electrically connected to a positive electrode and a negative electrode of the battery 22.

The heating device 24 includes a liquid absorbing member 241 and a heating member 242 electrically connected to the battery 22. The heating member 242 is wrapped up by the liquid absorbing member 241 or the liquid absorbing member 241 is wrapped up by the heating member 242. The liquid absorbing member 241 is disposed on the upper end of the supporting base 231. The liquid absorbing member 241 can be made of materials having a good absorbing performance, such as glass fiber or natural fiber, etc. Specifically, one end of the heating member 242 is connected to one of the electrode fixing bracket 232, and an opposite end of the heating member 242 is connected to the other electrode fixing bracket 232. The heating member 242 can be made of nichrome wire.

Referring to FIG. 9, the atomizing end cover 25 substantially has a cylindrical structure with flanges and two opposite ends are in communication with each other. The atomizing end cover 25 is provided with a second restricting protrusion 251 corresponding to the second restricting groove 2314. The second restricting protrusion 251 protrudes from one end of the atomizing end cover 25 away from the cartridge 10 along an axial direction of the atomizing end cover 25. The atomizing end cover 25 is provided, corresponding to the connecting body 132, with a fourth mounting hole 252 at a center of one end of the atomizing end cover 25 adjacent to the cartridge 10. The atomizing end cover 25 is provided with a third liquid inlet hole 253 around the fourth mounting hole 252. The outer wall of the atomizing end cover 25 is provided with an external thread 254 for engaging with the internal thread 145 of the adjusting member 14.

In assembling, the atomizing end cover 25 latches with the atomizing base 23 via the second restricting protrusion 251. An atomizing chamber 255 is formed by a space between the atomizing end cover 25 and the atomizing base 23. The heating device 24 is received in the atomizing chamber 255.

Referring to FIG. 10, in the embodiment, the controlling device includes a pressure sensor 26 disposed in the housing 21 and a controller (not shown). One end of the controller is connected to the pressure sensor 26, an opposite end of the controller is connected to the battery 22. The pressure sensor 26 is in communication with the air intake hole 211. When the user inhales, a negative pressure is generated in the housing 21. The pressure sensor 26 can detect the change of air pressure in the housing 21 and sends a control signal to the controller, such that the controller controls the battery 22 to supply power to the heating member 242 of the heating device 24, to realize powering on and heating automatically.

It can be understood that, in alternative embodiments, the controlling device can also be a switch (not shown) mounted on the outer wall of the housing 21. One end of the switch is connected to an electrode of the battery 22, and an opposite end of the switch is connected to the electrode fixing bracket 232. The switch can control the ON/OFF state of the battery 22 and the heating device 24. Specifically, the user sucks one puff, the switch is toggled one time, the battery 22 provides power to the heating device 24 one time.

The operation procedure of the electronic cigarette 100 of the present application is described below with reference to the accompanying drawings.

When the electronic cigarette 100 of the present application leaves factory, the cartridge 10 and the atomizing assembly 20 are each installed in place. The cartridge 10 and the atomizing assembly 20 are only required to be aligned and connected together before the electronic cigarette 100 can be used.

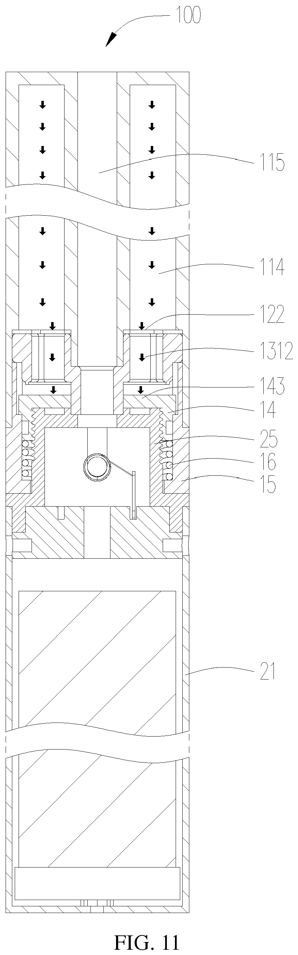

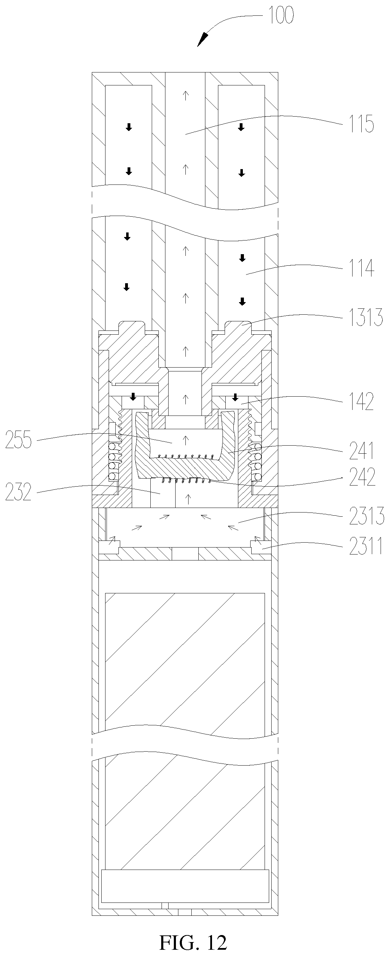

Referring simultaneously to FIG. 11 and FIG. 12, in assembling the cartridge 10, the cartridge 10 or the atomizing assembly 20 is rotated, and a screwing force is greater than an elastic force of the elastic member 16. The external thread 254 of the atomizing end cover 25 is screwed with the internal thread 145 of the adjusting member 14. Since the position of the atomizing base 23 stays unchanged, an axial movement of the atomizing end cover 25 is restricted, causing the adjusting member 14 to slide along the guiding groove 151 of the cartridge base 15 and move towards the atomizing end cover 25 along the axial direction of the cartridge base 15, and the elastic member 16 is compressed. At this time, the second liquid inlet hole 1312 of the liquid inlet member 13 is opened by the sealing portion 143 of the adjusting member 14. The tobacco liquid in the liquid storage chamber 114 passes through the first liquid inlet hole 122 and the second liquid inlet hole 1312, then passes through the communication portion 142 of the adjusting member 14, and finally flows into the atomizing chamber 255 of the atomizing end cover 25 via the third liquid inlet hole 253, to be absorbed by the liquid absorbing member 241 and atomized into aerosol under heating of the heating member 242. The thick arrows in FIG. 10 and FIG. 11 indicate a flowing direction of the tobacco liquid.

At the same time, the cartridge 10 is fixedly connected to the atomizing assembly 20 via an engagement between the internal thread 145 and the external thread 254, to avoid a disengagement of the cartridge 10 and the atomizing assembly 20.

When the user inhales, the air enters the electronic cigarette 100 via the air intake hole 211 of the housing 21 and passes sequentially through the air intake groove 2311 and the elongated groove 2313 of the atomizing base 23 to enter the atomizing chamber 255. The air mixed with the aerosol passes through the connecting body 132 of the liquid inlet member 13 and finally enters the user's mouth via the exhaust passage 115. The thin arrows in the FIG. 12 indicate a flowing direction of the air.

It can be understood that, the connecting body 132 can be omitted when the exhaust tube 113 is prolonged, to enable the exhaust tube 113 to sequentially extend through the pressing plate 12, the liquid inlet member 13, the adjusting member 14 and the atomizing end cover 25, causing the exhaust passage 115 to be directly in communication with the atomizing chamber 255.

In disassembling the cartridge 10, the cartridge 10 or the atomizing assembly 20 is rotated in a reversed direction. Due to the engagement between the external thread 254 of the atomizing end cover 25 and the internal thread 145 of the adjusting member 14, the adjusting member 14 slides along the guiding groove 151 of the atomizing base 23 and moves in a direction away from the atomizing end cover 25, and the elastic member 16 resets and finally pushes the adjusting member 14 against the liquid inlet member 13 tightly. The sealing portion 143 of the adjusting member 14 seals the second liquid inlet hole 1312 of the liquid inlet member 13, to avoid a leakage of the tobacco liquid. Even if the children contact and open the electronic cigarette, it will not cause harm to the children.

In the present application, only when the electronic cigarette is assembled for use, the tobacco liquid flows out to be atomized, but after the electronic cigarette is disassembled, the tobacco liquid is immediately sealed. Thus, it has a good protection function for children with a simple structure and is convenient to operate.

The embodiments described above are merely preferred embodiments, not intended to limit the application. Any modifications, alternatives or improvements made within the principle of the present application should be interpreted as falling within the protection scope of the present application.

## Claims

1. A cartridge comprising a cartridge carrier (11) having a liquid storage chamber (114), a liquid inlet member (13) and an adjusting member (14) received in the cartridge carrier (11), a cartridge base (15) disposed at one end of the cartridge carrier (11), and an elastic member (16) received in the cartridge base (15), wherein the liquid inlet member (13) is provided with a second liquid inlet hole (1312) in communication with the liquid storage chamber (114), the adjusting member (14) is provided with a communication portion (142) and a sealing portion (143), the adjusting member (14) is slidably disposed between the liquid inlet member (13) and the cartridge base (15), one end of the elastic member (16) elastically resists the adjusting member (14), the other end of the elastic member (16) elastically resists the cartridge base (15) to cause the sealing portion (143) to seal the second liquid inlet hole (1312), when the adjusting member (14) is brought to slide in a direction away from the liquid inlet member (13), the communication portion (142) is in communication with the second liquid inlet hole (1312).

2. The cartridge according to claim **1,** wherein an outer wall of the adjusting member (14) is provided with at least one guiding protrusion (144) along an axial direction of the adjusting member (14), and an inner wall of the cartridge base (15) is provided, corresponding to the guiding protrusion (144), with at least one guiding groove (151) along an axial direction of the cartridge base (15), or an outer wall of the adjusting member (14) is provided with at least one guiding groove (151) along an axial direction of the adjusting member (14), and an inner wall of the cartridge base (15) is provided, corresponding to the guiding groove (151), with at least one guiding protrusion (144) protruding inwardly along an axial direction of the cartridge base (15), the guiding protrusion (144) is capable of sliding along the guiding groove (151).

3. The cartridge according to claim **2,** wherein the inner wall of the cartridge base (15) is provided with an abutting portion (152) at one end away from the cartridge carrier (11), the abutting portion (152) protrudes radially inwardly along a circumferential direction of the cartridge base (15), one end of the elastic member (16) elastically resists the abutting portion (152), the other end of the elastic member (16) elastically resists the adjusting member (14) at one end away from the liquid inlet member (13).

4. The cartridge according to any one of claims **1** to **3,** wherein the cartridge carrier (11) comprises a liquid storage tube (112) and an exhaust tube (113) extending through the liquid storage tube (112) along an axial direction of the liquid storage tube (112), the liquid storage chamber (114) is formed by a space between an inner wall of the liquid storage tube (112) and an outer wall of the exhaust tube (113), an exhaust passage (115) is formed by an interior space of the exhaust tube (113).

5. The cartridge according to claim **4,** further comprising a pressing plate (12) received in the cartridge carrier (11), wherein the pressing plate (12) is fixedly mounted on one end of the liquid inlet member (13) away from the adjusting member (14), the pressing plate (12) is provided with a first liquid inlet hole (122) corresponding to the second liquid inlet hole (1312), the first liquid inlet hole (122) is in communication with the liquid storage chamber (114).

6. An atomizer comprising the cartridge (10) according to any one of claims **1** to **5,** wherein the atomizer further comprises an atomizing assembly (20) detachably connected to the cartridge (10), the atomizing assembly (20) comprises an atomizing end cover (25) connected to the adjusting member (14) by threads, when the cartridge (10) or the atomizing assembly (20) is rotated, the adjusting member (14) is driven to move towards or away from the liquid inlet member (13).

7. The atomizer according to claim **6,** wherein an inner wall of the adjusting member (14) is provided with an internal thread (145), an outer wall of the atomizing end cover (25) is provided with an external thread (254) matching with the internal thread (145), the external thread (254) of the atomizing end cover (25) extends through the cartridge base (15) and engages with the internal thread (145).

8. The atomizer according to claim **6,** wherein an atomizing chamber (255) is formed by an interior space of the atomizing end cover (25), the atomizing end cover (25) is provided with a third liquid inlet hole (253) in communication with the atomizing chamber (255).

9. The atomizer according to claim **8,** wherein the pressing plate (12) is provided with a first mounting hole (121), the liquid inlet member (13) is provided with a second mounting hole (1311), the adjusting member (14) is provided with a third mounting hole (141), the atomizing end cover (25) is provided with a fourth mounting hole (252), the exhaust tube (113) extends sequentially through the first mounting hole (121), the second mounting hole (131), the third mounting hole (1411) and the fourth mounting hole (252), to enable the atomizing chamber (255) to be in communication with the exhaust passage (115).

10. An electronic cigarette comprising the atomizer according to any one of claims **6** to **9,** wherein the electronic cigarette (100) further comprises a battery assembly connected to the atomizer.
